# EUROPEAN PATENT APPLICATION

(11) **EP 2 818 846 A1**
(43) Date of publication of application: **31.12.2014**
(21) Application number: 12869106.0
(22) Date of filing: 30.11.2012
(51) Int. Cl.: G01N 15/06, G01N 15/00, G01N 21/64

(54) **PARTICLE DETECTOR AND AIR-CONDITIONER**

(30) Priority: 23.02.2012 JP 2012037324
(71) Applicant: Sharp Kabushiki Kaisha, Osaka-shi, Osaka 545-8522 (JP)
(72) Inventor: FUJITA, Hideaki, Osaka-shi OSAKA 545-8522 (JP); KAMIYAMA, Haruki, Osaka-shi OSAKA 545-8522 (JP); KAMO, Tomonori, Osaka-shi OSAKA 545-8522 (JP); SUZUKI, Akihiro, Osaka-shi OSAKA 545-8522 (JP)
(74) Representative: Müller-Boré & Partner Patentanwälte PartG mbB
(86) International application number: PCT/JP2012/081078
(87) International publication number: WO 2013/125121

(57) **Abstract**

There is provided a particle detector that detects biogenic particles with high sensitivity. The particle detector includes a collecting member 10 having a principal surface and configured to electrostatically collect particles on the principal surface, an irradiation unit 21 configured to irradiate the particles collected on the principal surface with excitation light, a light receiving unit 34 configured to receive fluorescence emitted from the particles by irradiation of the particles with the excitation light, and a detection unit configured to detect biogenic particles from the particles collected on the principal surface on the basis of a fluorescence intensity in the light receiving unit. The particle detector further includes a filter 37 disposed between the principal surface and the light receiving unit cut light with a wavelength emitted by irradiation of the excitation light from a substance that is generated on the principal surface when the particles are electrostatically collected.

## Description

### Technical Field

The present invention relates to a particle detector and an air conditioner, and more particularly, to a particle detector and an air conditioner that detect biogenic particles.

### Background Art

As a device for counting microorganisms serving as biogenic particles, for example, Japanese Unexamined Patent Application Publication No. 2008-1287935 (hereinafter referred to as PTL 1) discloses a technique in which microorganisms are fixed on a surface of a collection means, such as a membrane filter, and are impregnated with a fluorescent staining reagent. A fluorochrome is irradiated with excitation light, such as laser light, and a fluorescent image of the surface is acquired, and the microorganisms are detected by extracting luminous points from the fluorescent image.

### Citation List

### Patent Literature

PTL 1: Japanese Unexamined Patent Application Publication No. 2008-187935

### Summary of Invention

### Technical Problem

In the method for detecting fluorescence from biogenic particles by irradiating the particles with excitation light, when the excitation light reaches a light receiving unit, it becomes noise and decreases fluorescence detection sensitivity of the light receiving unit. Even when a laser light source is used as a light source as in PTL 1, a component to become noise exists. The noise component affects the fluorescence detection sensitivity particularly in high-sensitivity measurement.

There is a limit to removal of such a noise component only by separating excitation light by the filter, and sufficient fluorescence detection sensitivity is sometimes not obtained. Particularly in high-sensitivity measurement, the detection accuracy is impaired.

The present invention has been made in view of such a problem, and an object of the invention is to provide a particle detector and an air conditioner that detect biogenic particles with high sensitivity.

### Solution to Problem

To achieve the above object, according to an aspect of the present invention, a particle detector includes a collecting member having a principal surface and configured to electrostatically collect particles on the principal surface, an irradiation unit configured to irradiate the particles collected on the principal surface with excitation light, a light receiving unit configured to receive fluorescence emitted from the particles collected on the principal surface by irradiation of the particles with the excitation light, and a detection unit configured to detect biogenic particles from the particles collected on the principal surface on the basis of a fluorescence intensity in the light receiving unit. The particle detector further includes a filter disposed between the principal surface and the light receiving unit to cut light with a wavelength emitted by irradiation of the excitation light from a substance that is generated on the principal surface when the particles are electrostatically collected.

Preferably, the principal surface is a mirror surface, and the fluorescence is specularly reflected by the principal surface.

Preferably, the particle detector further includes a heating unit configured to heat the particles, and the filter cuts light with a wavelength of fluorescence from a substance whose fluorescence amount changes between before and after heating with the heating unit, of the substance generated when the particles are electrostatically collected.

Preferably, the filter cuts light with a wavelength of fluorescence from a nitric acid compound.

Preferably, a cutoff wavelength λi of the filter is longer than or equal to 650 nm.

Preferably, the light receiving unit includes a light collecting lens, and the light collecting lens contains a material that absorbs the excitation light. According to another aspect of the present invention, an air conditioner includes the above-described particle detector and a display unit, and displays, on the display unit, a detection result of a number of particles in the air detected by the particle detector.

### Advantageous Effects of Invention

According to the present invention, it is possible to detect biogenic particles with high sensitivity.

### Brief Description of Drawings

[Fig. 1] Fig. 1 includes graphs showing the change in fluorescence intensity of biogenic particles between before and after heating and the change in fluorescence intensity of powder dust between before and after heating.
[Fig. 2] Fig. 2 illustrates a collection step for detecting biogenic particles.
[Fig. 3] Fig. 3 illustrates a fluorescence measuring step (before heating) for detecting the biogenic particles.
[Fig. 4] Fig. 4 illustrates a heating step for detecting the biogenic particles.
[Fig. 5] Fig. 5 illustrates a fluorescence measuring step (after heating) for detecting the biogenic particles.
[Fig. 6] Fig. 6 illustrates a refresh step for detecting the biogenic particles.
[Fig. 7] Fig. 7 is a graph showing the relationship between an increase amount ΔF of fluorescence intensity between before and after heating and the concentration of biogenic particles.
[Fig. 8] Fig. 8 explains a detection state in an ideal condition in a particle detector according to an embodiment.
[Fig. 9] Fig. 9 shows the relationship between the increase in stray light due to cleaning residues and the dynamic range of fluorescence intensity.
[Fig. 10] Fig. 10 is a micrograph of a surface of a collecting substrate on which cleaning residues are left before heating.
[Fig. 11] Fig. 11 is a micrograph of the surface of the collecting substrate on which cleaning residues are left after heating.
[Fig. 12] Fig. 12 shows the relationship between the change in stray light amount due to cleaning residues between before and after heating and measurement error of fluorescence intensity.
[Fig. 13] Fig. 13 is a graph showing a specific example of a filter characteristic of a long pass filter.
[Fig. 14] Fig. 14 is an enlarged view of a portion where the optical spectral intensity of excitation light shown by curve (2) of Fig. 13 is low.
[Fig. 15] Fig. 15 is a perspective view illustrating an appearance of the particle detector according to the embodiment.

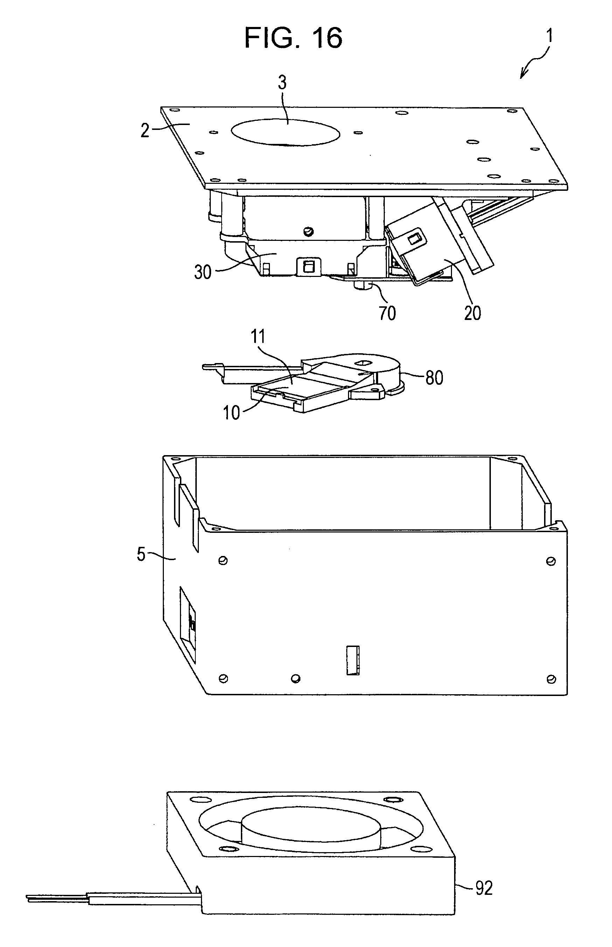

[Fig. 16] Fig. 16 is a perspective view illustrating an exploded state of the particle detector illustrated in Fig. 15.
[Fig. 17] Fig. 17 is an exploded perspective view illustrating a detailed structure of the particle detector.
[Fig. 18] Fig. 18 is a cross-sectional view of the particle detector.
[Fig. 19] Fig. 19 is a schematic view illustrating the behavior of light in an optical system included in the particle detector.
[Fig. 20] Fig. 20 is a schematic view illustrating light reflection on a principal surface of a substrate.
[Fig. 21] Fig. 21 is a graph showing a specific example of a filter characteristic of a filter included in an excitation optical system of the particle detector.
[Fig. 22] Fig. 22 is a schematic view of a cross section of a Fresnel lens.
[Fig. 23] Fig. 23 is a graph showing a specific example of a filter characteristic of a filter included in a light receiving optical system of the particle detector.
[Fig. 24] Fig. 24 is a flowchart showing the flow of operations of the particle detector.
[Fig. 25] Fig. 25 shows the time change of a fluorescence spectrum of Penicillium between before and after heat treatment.
[Fig. 26] Fig. 26 shows a fluorescence spectrum of fluorescence-emitting dust before heat treatment.
[Fig. 27] Fig. 27 shows a fluorescence spectrum of the fluorescence-emitting dust after heat treatment.
[Fig. 28] Fig. 28 illustrates a specific example of an appearance of an air purifier including the particle detector.
[Fig. 29] Fig. 29 is a block diagram showing a specific example of a functional configuration of the air purifier.
[Fig. 30] Fig. 30 is a graph showing results of measurement of the amount of stray light in three states, that is, a state in which none of the filter included in the excitation optical system and the filter included in the light receiving optical system are provided (state 1), a state in which only the former filter is provided, but the latter filter is not provided (state 2), and a state in which both of the filters are provided (state 3).
[Fig. 31] Fig. 31 is a graph showing results of measurement of the fluorescence intensity when a predetermined amount of Penicillium is collected on a substrate 10 in three states, that is, the state in which none of the filter included in the excitation optical system and the filter included in the light receiving optical system are provided (state 1), the state in which only the former filter is provided, but the latter filter is not provided (state 2), and the state in which both of the filters are provided (state 3).
[Fig. 32] Fig. 32 shows filter characteristics of four types of IR (infrared) filters serving as the filter included in the light receiving optical system.
[Fig. 33] Fig. 33 is a graph showing results of measurement of the amount of stray light and the fluorescence intensity when the four types of filters shown in Fig. 32 are used.

### Description of Embodiments

An embodiment of the present invention will be described below with reference to the drawings. In the following description, the same components and constituent element are denoted by the same reference numerals. The same components and constituent element have the same names and the same functions.

### [Regarding Detection Principle of Biogenic Particles]

A particle detector according to the embodiment is a device that detects biogenic particles such as pollen, microorganisms, and mold. First, a description will be given of the principle on which biogenic particles are detected with the particle detector of the embodiment.

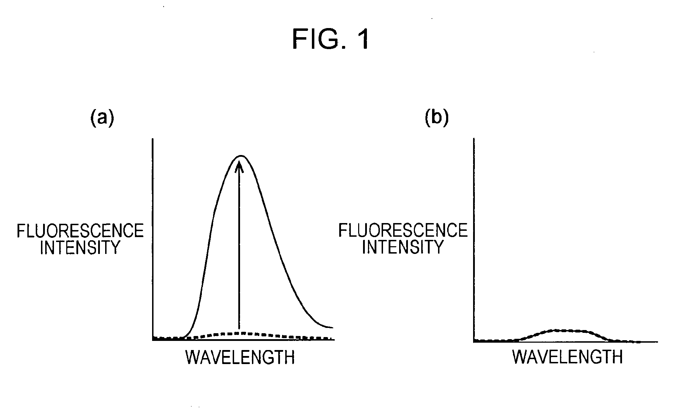

Fig. 1 includes graphs showing the change in fluorescence intensity of biogenic particles between before and after heating and the change in fluorescence intensity of powder dust between before and after heating.

When biogenic particles suspended in the air is irradiated with ultraviolet light or blue light, they emit fluorescence. However, particles that similarly emit fluorescence, such as lint of chemical fiber (hereinafter also referred to powder dust), are suspended in the air. Hence, when only the fluorescence is detected, it is not determined whether the fluorescence is emitted from the biogenic particles or the powder dust.

On the other hand, when heat treatment is conducted on biogenic particles and powder dust and the changes in fluorescence intensity (fluorescence amount) between before and after heating are measured, as shown in Fig. 1, the intensity of fluorescence emitted from the powder dust is not changed by the heat treatment, as shown by Fig. 1(b), whereas the intensity of fluorescence emitted from the biogenic particles is increased by the heat treatment, as shown in Fig. 1(a). In the particle detector of the embodiment, the fluorescence intensity of particles, in which biogenic particles and powder dust are mixed, is measured before and after heating, and the difference therebetween is obtained to specify the number of biogenic particles.

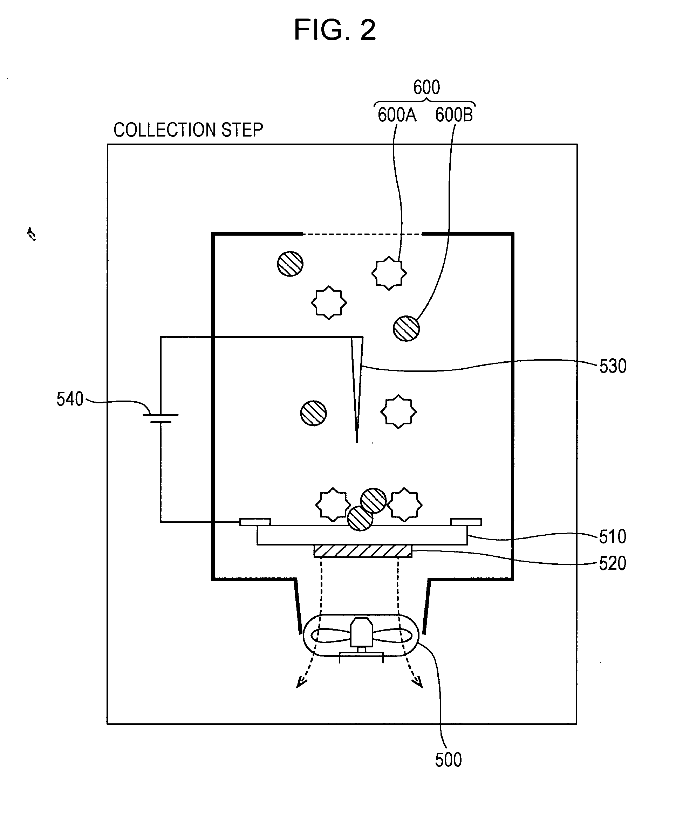

Figs. 2 to 6 illustrate steps for detecting biogenic particles. Referring to Fig. 2, first, particles are collected on a collecting substrate 510 (collection step).

In this step, the collecting substrate 510 is placed opposed to an electrostatic probe 530, and a potential difference is generated between the collecting substrate 510 and the electrostatic probe 530. When air is introduced toward the collecting substrate 510 by driving a fan 500, particles 600 suspended in the air are charged around the electrostatic probe 530. The charged 600 are attracted onto a surface of the collecting substrate 510 by electrostatic force. The particles 600 collected on the collecting substrate 510 contain biogenic particles 600A and powder dust 600B such as lint of chemical fiber.

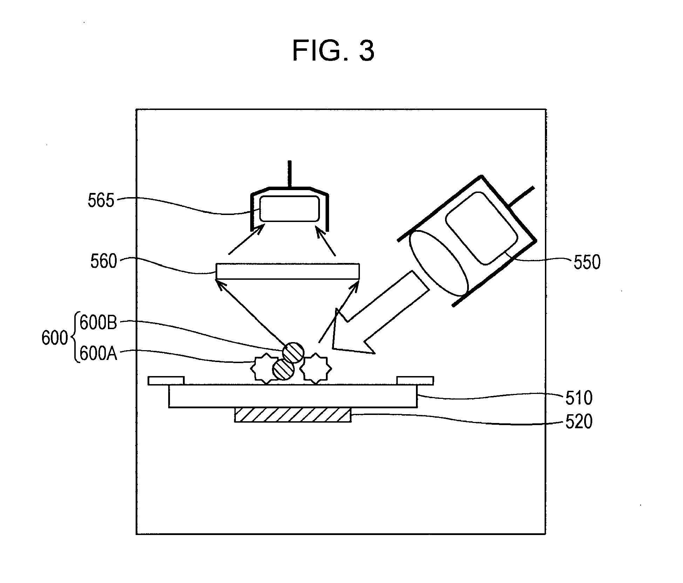

Next, as illustrated in Fig. 3, the intensity of fluorescence emitted from the particles 600 before heating is measured. In this step, excitation light is applied from a light emitting element 550, such as a semiconductor laser, toward the particles 600 collected on the collecting substrate 510, and fluorescence emitted from the particles 600 is received by a light receiving element 565 via a lens 560.

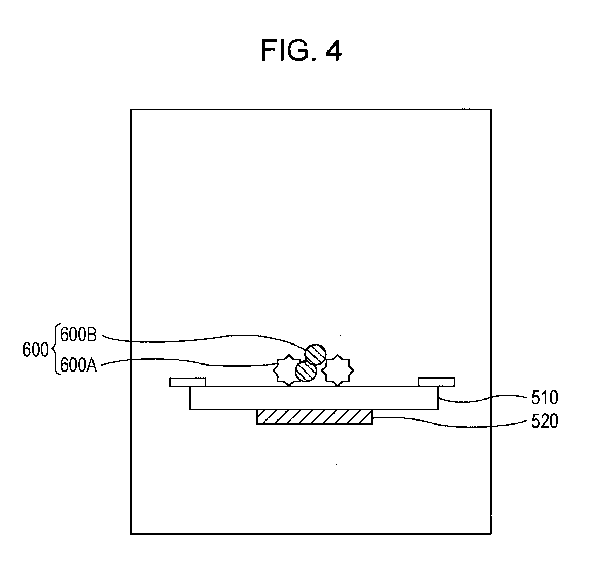

Next, as illustrated in Fig. 4, the particles 600 collected on the collecting substrate 510 are heated by a heater 520. After heating, the collecting substrate 510 is cooled.

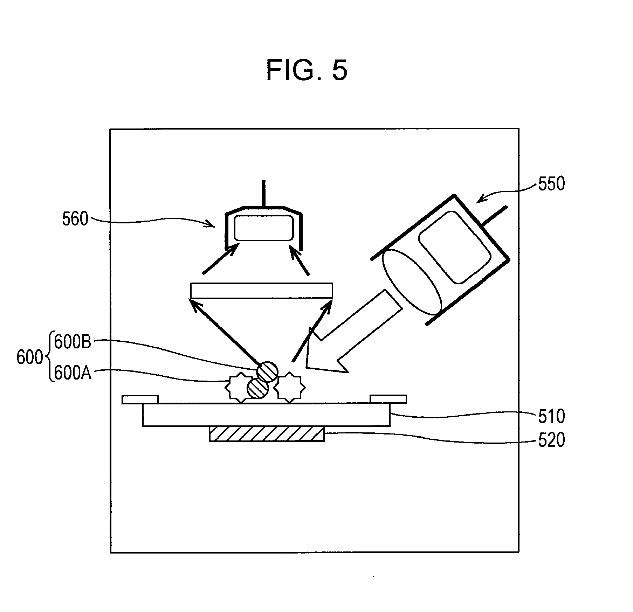

Next, as illustrated in Fig. 5, the intensity of fluorescence emitted from the particles 600 after heating is measured. As described above, the intensity of fluorescence emitted from the powder dust 600B is not changed by heat treatment, whereas the intensity of fluorescence emitted from the biogenic particles 600A is increased by heat treatment. For this reason, the fluorescence intensity measured in this step is higher than the fluorescence intensity measured before heating in the fluorescence measuring step illustrated in Fig. 3.

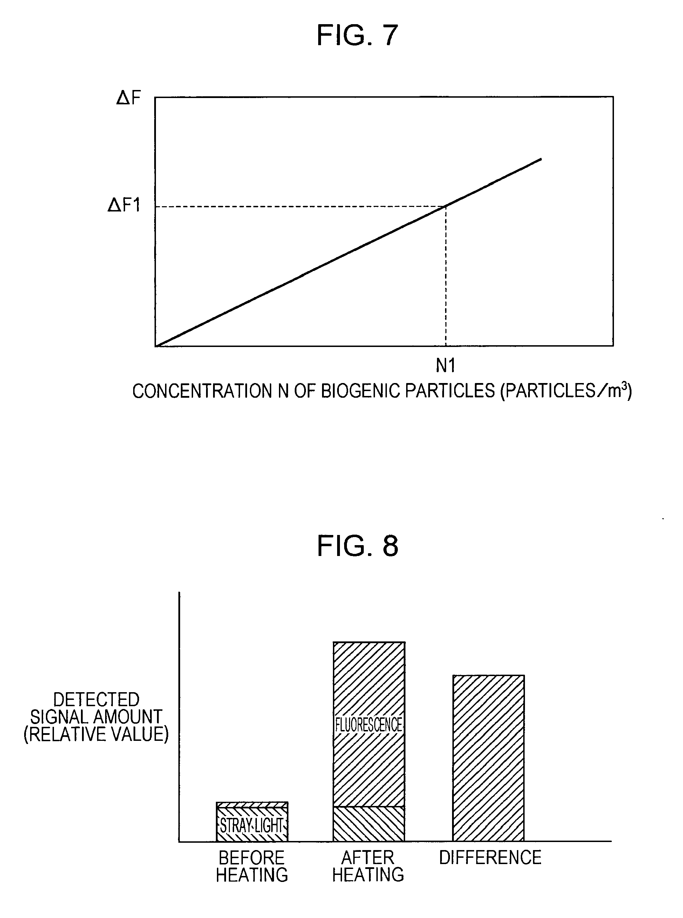

Fig. 7 is a graph showing the relationship between an increase amount ΔF of fluorescence intensity between before and after heating and the concentration of biogenic particles. Referring to Fig. 7, an increase amount ΔF1 of fluorescence intensity is calculated from a difference between the fluorescence intensity before heating and the fluorescence intensity after heating. On the basis of a prepared relationship between the increase amount ΔF of fluorescence intensity and a biogenic particle concentration N, a biogenic particle concentration N1 corresponding to the calculated increase amount ΔF1 is specified. The correspondence relationship between the increase amount ΔF and the biogenic particle concentration N is experimentally determined beforehand.

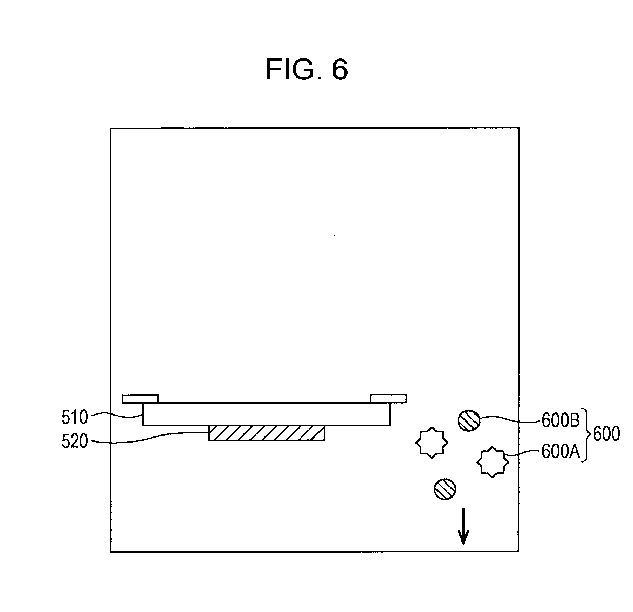

Fig. 6 illustrates the following refresh step. In the refresh step, after detection of the biogenic particles is finished, the particles 600 are removed from the collecting substrate 510 by being raked off by an unillustrated brush.

### [Description of Problems]

The particle detector of the embodiment detects biogenic particles, such as pollen, microorganisms, and mold, by utilizing the above-described detection principle. At that time, irradiation light from the light emitting element 550, such as a semiconductor laser, contains a noise component, and the noise component is included as stray light in the intensity of light received by the light receiving element 565.

Fig. 8 explains a detection state in an ideal condition. That is, referring to Fig. 8, in an ideal condition, a stray light component is removed by eliminating the difference between before and after heating, and only an increase in fluorescence intensity between before and after heating is detected.

However, if the particles 600 are not completely removed from the collecting substrate 510 by cleaning with the unillustrated brush in the refresh step illustrated in Fig. 6, residues accumulate on the surface of the collecting substrate 510.

A first problem occurs in that stray light increases as cleaning residues increase. With the increase in stray light, the dynamic range of the fluorescence intensity decreases.

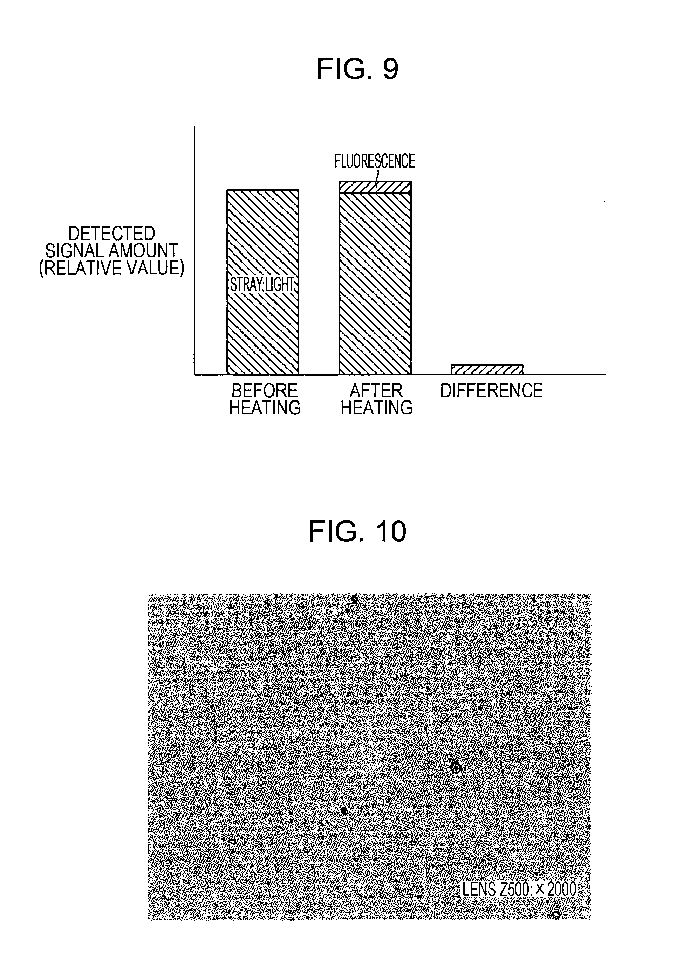

Fig. 9 shows the relationship between the increase in stray light due to cleaning residues and the dynamic range of the fluorescence intensity. Referring to Fig. 9, when stray light increases owing to the cleaning residues, the dynamic range of the detected fluorescence intensity is reduced and becomes insufficient to detect the fluorescence intensity. Thus, the measurement accuracy of the fluorescence intensity decreases.

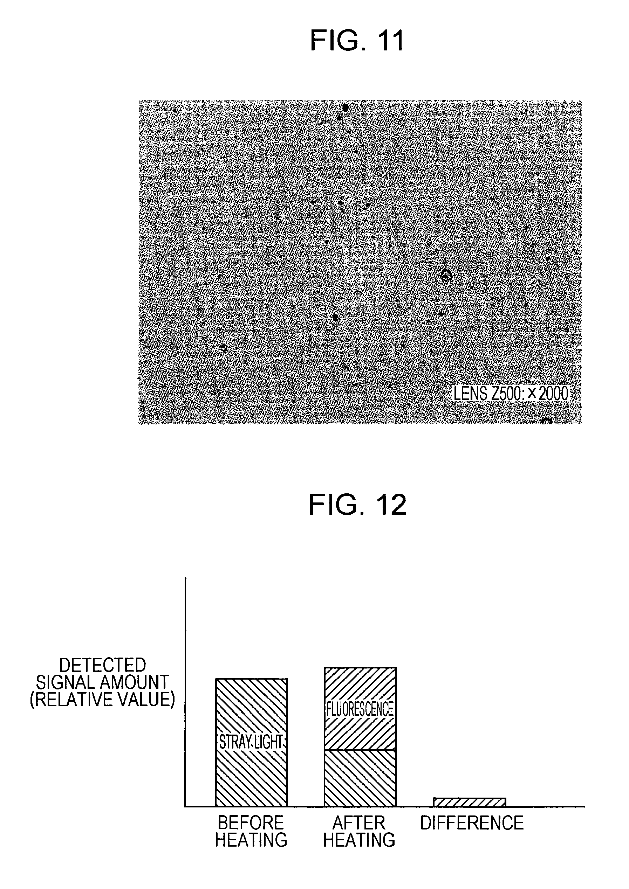

The size (shape) of cleaning residues is sometimes changed by heating. Figs. 10 and 11 are micrographs of the surface of the collecting substrate 510 on which cleaning residues are left before and after heating, respectively. Figs. 10 and 11 show that the particle size (shape) of the cleaning residues is changed by heating. For this reason, a second problem occurs in that the amount of stray light is changed by the cleaning residues between before and after heating. The change in amount of stray light between before and after heating increases measurement error of the fluorescence intensity.

Fig. 12 shows the relationship between the change in amount of stray light due to cleaning residues between before and after heating and the measurement error of the fluorescence intensity. Referring to Fig. 12, when the amount of stray light after heating decreases to be less than the amount of stray light before heating, measurement error occurs, that is, the increase amount in fluorescence intensity is cancelled by the decrease, and the increase amount in fluorescence is measured as an increase amount smaller than an actual increase amount.

For example, the easiest method for solving the first problem and the second problem described above is to remove cleaning residues. Although performance of the brush needs to be enhanced for that purpose, it is difficult to completely remove the cleaning residues, because not only particles are collected but also nitric acid and a nitric acid compound are generated on the collecting substrate owing to ozone at the time of electrostatic collection.

Accordingly, to reduce the influence of cleaning residues on the detection accuracy, the particle detector of the embodiment includes an optical system such that stray light is not generated even when cleaning residues are left on the collecting substrate.

For example, a first cause of occurrence of stray light due to cleaning residues is scattering of excitation light by irregularities of the cleaning residues.

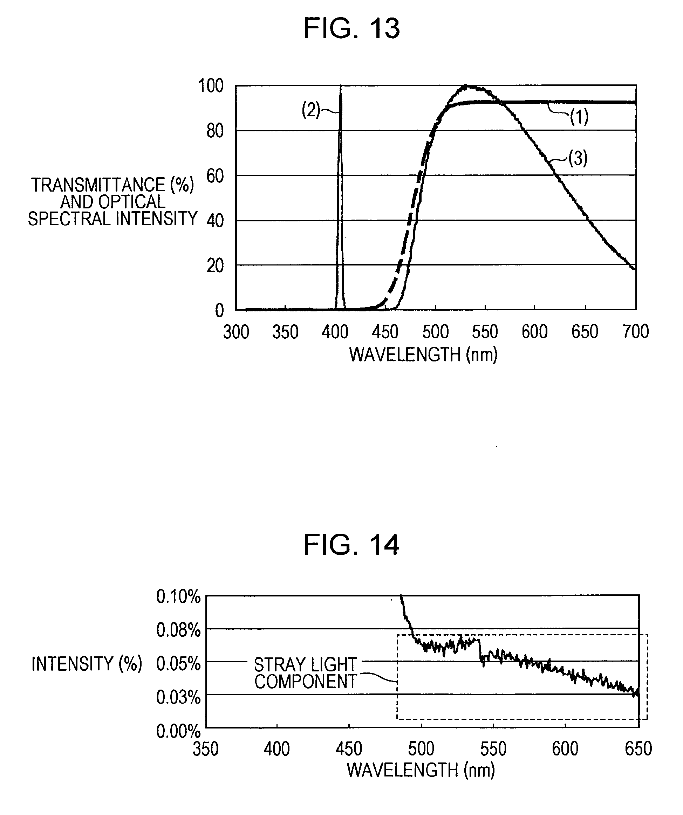

To deal with the above first cause, it is conceivable to dispose a long pass filter in front of the light receiving element to block excitation light to be incident on the light receiving element. Fig. 13 is a graph showing a specific example of a filter characteristic of the long pass filter. The horizontal axis in the graph of Fig. 13 indicates the wavelength of light. The unit of the wavelength is nm. The vertical axis in the graph of Fig. 13 indicates the transmittance of the filter and the spectral intensity of light. The unit of transmittance of the filter is %. The spectral intensity of light refers to the relative value of spectral intensity at each wavelength when the wavelength such that the spectral intensity of excitation light or fluorescence becomes the highest is 100. In Fig. 13, curve (1) shows the characteristic of the long pass filter, curve (2) shows the spectral intensity of excitation light from the semiconductor laser element, and curve (3) shows the spectral intensity of fluorescence emitted from Penicillium irradiated with the excitation light.

According to the filter characteristic shown by curve (1), the long pass filter has the property of blocking most of light with a wavelength of about 440 nm or less and transmitting most of light with a wavelength of about 500 nm or more. The long pass filter functions as a high-pass filter (or a low-cut filter) that blocks light with a wavelength shorter than or equal to a predetermined threshold value and transmits light with a wavelength longer than or equal to the threshold value.

As shown by curve (2), excitation light from the semiconductor laser has the peak value of optical spectral intensity at a wavelength of about 400 nm. In contrast, as shown by curve (3), the spectrum of fluorescence F from Penicillium is measured in a wavelength range longer than or equal to about 460 nm, and the fluorescence F has the peak value of optical spectral intensity at a wavelength of about 530 nm.

By using the long pass filter having the filter characteristic shown by curve (1), most of the excitation light having the optical spectral intensity shown by curve (2) is absorbed by the long pass filter, and incidence thereof on the light receiving element is almost blocked. In contrast, the fluorescence having the optical spectral intensity shown by curve (3) enters the light receiving element while mostly maintaining the optical spectral intensity.

Fig. 14 is an enlarged view of a portion of the excitation light where the optical spectral intensity shown by curve (2) of Fig. 13 is low. As shown in Fig. 14, the excitation light from the semiconductor laser shown by curve (2) contains a faint and low component on a long wavelength side. This faint and long-wavelength component contained in the excitation light is considered to be a main cause of stray light because it is not blocked by the long pass filter having the filter characteristic of curve (1).

Accordingly, in the particle detector of the embodiment, incidence of the long-wavelength component of the excitation light on the light receiving element is blocked by utilizing a low pass filter (or a high cut filter) to deal with the above first cause.

Next, emission of fluorescence from cleaning residues irradiated with excitation light is given as a second cause of the occurrence of stray light due to the cleaning residues. As described above, when particles are collected according to the above-described principle, high voltage is applied between the collecting substrate 510 and the electrostatic probe 530, corona discharge is caused, and ozone is generated. When the ozone is generated, nitrogen oxide is generated, and the nitrogen oxide reacts with moisture in the air, so that nitric acid is generated. The generated nitric acid adheres to the collecting substrate. Alternatively, the nitric acid is accumulated on the collecting substrate as a nitric acid compound such as nitrate. It is known that the nitric acid and the nitric acid compound are phosphors and emit fluorescence containing many infrared components when irradiated with laser light.

Accordingly, in the particle detector of the embodiment, to deal with the above-described first cause, an IR (infrared) cut filter is provided in front of the light receiving element to block incidence of infrared components on the light receiving element.

### [Overall Configuration of Particle Detector]

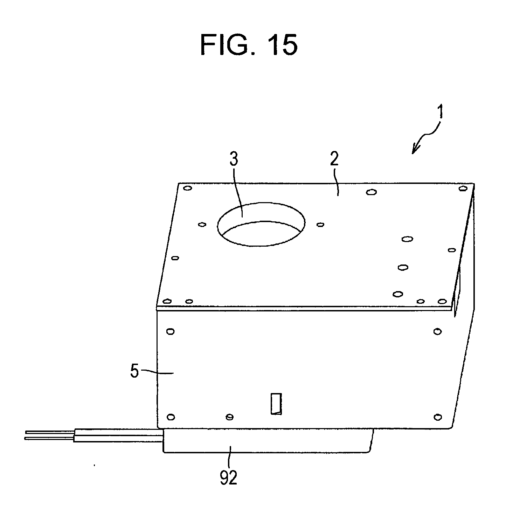

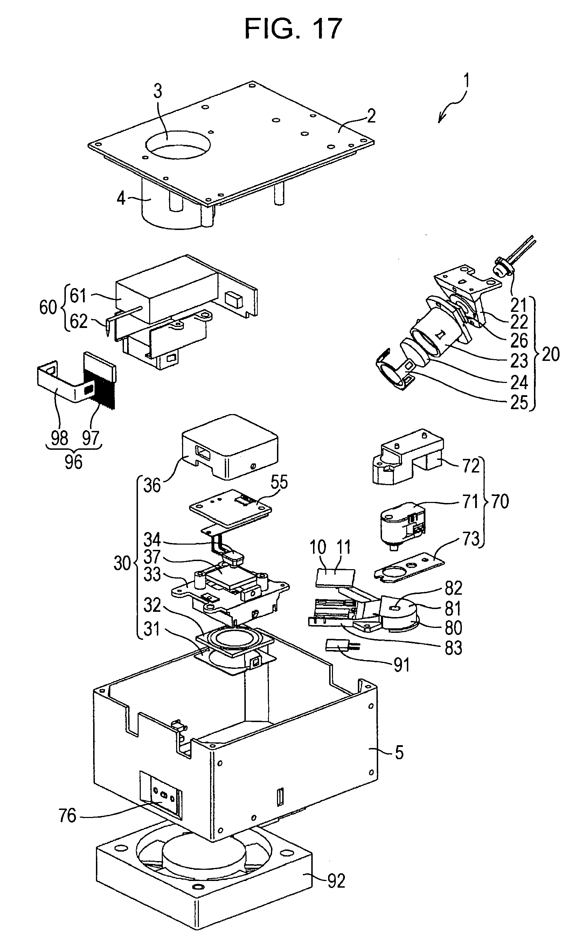

Fig. 15 is a perspective view illustrating an appearance of a particle detector 1 according to the embodiment. Fig: 16 is a perspective view illustrating an exploded state of the particle detector 1 of Fig. 1. Fig. 17 is an exploded perspective view illustrating a detailed structure of the particle detector 1. The particle detector 1 of the embodiment is a device that detects biogenic particles such as pollen, microorganisms, and mold.

The particle detector 1 of the embodiment includes an upper cabinet 2 serving as a first housing, and a lower cabinet 5 serving as a second housing. The upper cabinet 2 is shaped like a substantially rectangular flat plate. The lower cabinet 5 is shaped like a substantially rectangular parallelepiped container having a bottom and opening in one direction. The upper cabinet 2 and the lower cabinet 5 are assembled such that the upper cabinet 2 closes an opening of the lower cabinet 5 like a lid, so that a hollow cabinet having an outer shape like a substantially rectangular parallelepiped is formed.

Devices that constitute the particle detector 1, such as a collecting unit 60, an excitation optical system 20, a light receiving optical system 30, and a cleaning unit 96, except for a fan 92, are contained in the cabinet. As an example, the cabinet has a size of 60 mm x 50 mm (length and width of the upper cabinet 2) x 30 mm (height).

A collecting cylinder 4 serving as a cylindrical member is provided integrally with the upper cabinet 2. The collecting cylinder 4 is shaped like a hollow cylinder, and extends from the upper cabinet 2 into the inside of the particle detector 1 while being attached at one end to a lower surface of the upper cabinet 2. The upper cabinet 2 has an introducing portion 3 that penetrates a part of the upper cabinet 2 in a thickness direction. The outside of the particle detector 1 and the inside of the collecting cylinder 4 communicate with each other via the introducing portion 3. The collecting cylinder 4 is provided to surround a below-described electrostatic probe 62. Air containing particles is introduced from the introducing portion 3 into the collecting cylinder 4. The collecting cylinder 4 guides the air containing particles toward a substrate 10 positioned opposed to the electrostatic probe 62.

A fan 92 is attached to an outer side of a bottom surface of the lower cabinet 5. The bottom surface of the lower cabinet 5 to which the fan 92 is attached has an aperture. This aperture opens to include an area opposed to the collecting cylinder 4 and an area opposed to a below-described brush 97, and is continuously formed by the area opposed to the collecting cylinder 4 and the area opposed to the brush 97.

The fan 92 can be rotationally driven in a forward direction and a reverse direction. When the fan 92 is driven in the forward direction, air in an inner space of the particle detector 1 is exhausted out of the particle detector 1 through the fan 92. When the fan 92 is driven in the reverse direction, air outside the particle detector 1 is introduced into the inner space of the particle detector 1 through the fan 92. The fan 92 is used for collection of particles into the particle detector 1, cooling of the particles after heating, and cleaning of the substrate 10 for collecting the particles. This reduces the size and cost of the particle detector 1.

The particle detector 1 includes a collecting unit 60. The collecting unit 60 collects particles contained in air onto a principal surface 11 of a substrate 10 serving as a collecting member. The collecting unit 60 includes a collection power-supply circuit 61 formed by a high-voltage power supply, and an electrostatic probe 62 serving as a discharge electrode. The substrate 10 has the principal surface 11. The substrate 10 is provided as a collecting member that collects, onto the principal surface 11, particles in which biogenic particles and powder dust, such as lint of chemical fiber, are mixed.

The substrate 10 is formed by a silicon flat plate. On the principal surface 11 of the substrate 10 for attracting particles, a conductive transparent coating is provided. The substrate 10 is not limited to silicon, but may be formed of glass, a ceramic material, or metal. The coating is not limited to the transparent coating, but, for example, a metallic coating may be provided on the surface of the substrate 10 formed of a ceramic material. When the substrate 10 is formed of metal, it is unnecessary to form a coating on the surface of the substrate 10. As long as the substrate 10 is a silicon substrate, the material itself is inexpensive, and it is easy to conduct mirror finishing on the principal surface 11 and to form the conductive coating on the principal surface 11.

The collection power-supply circuit 61 is provided as a power supply part that produces a potential difference between the substrate 10 and the electrostatic probe 62. The electrostatic probe 62 extends from the collection power-supply circuit 61, penetrates the collecting cylinder 4, and reaches an inner portion of the collecting cylinder 4. The substrate 10 is disposed opposed to the electrostatic probe 62. In this embodiment, the electrostatic probe 62 is electrically connected to a positive electrode of the collection power-supply circuit 61. The coating provided on the principal surface 11 of the substrate 10 is electrically connected to a negative electrode of the collection power-supply circuit 61.

The electrostatic probe 62 may be electrically connected to the positive electrode of the collection power-supply circuit 61, and the coating provided on the substrate 10 may be connected to a ground potential. Alternatively, the electrostatic probe 62 may be electrically connected to the negative electrode of the collection power-supply circuit 61, and the coating provided on the substrate 10 may be electrically connected to the positive electrode of the collection power-supply circuit 61.

When the fan 92 is driven in the forward direction, air in the cabinet of the particle detector 1 is exhausted, and simultaneously, air outside the cabinet is introduced toward the substrate 10 through the collecting cylinder 4. At this time, when a potential difference is produced between the electrostatic probe 62 and the substrate 10 by the collection power-supply circuit 61, particles in the air are positively charged around the electrostatic probe 62. The positively charged particles are moved to the substrate 10 by electrostatic force, are attracted on the conductive coating provided on the principal surface 11 of the substrate 10, and are thereby collected on the substrate 10.

In this way, in the particle detector 1 of the embodiment, particles are collected on the substrate 10 by charge collection utilizing electrostatic force. In this case, it is possible to reliably hold the particles on the substrate 10 during detection of the particles and to easily remove the particles from the substrate 10 after detection of the particles. By using the needle-like electrostatic probe 62 as a discharge electrode, the charged particles can be attracted on the surface of the substrate 10 opposed to the electrostatic probe 62 and in an extremely narrow region corresponding to an irradiation region of a below-described light emitting element. This allows attracted microorganisms to be efficiently detected in the fluorescence measuring step.

The particle detector 1 includes an excitation optical system 20 and a light receiving optical system 30. The excitation optical system 20 functions as a light irradiation unit that irradiates particles collected on the principal surface 11 of the substrate 10 with excitation light. The light receiving optical system 30 functions as a light receiving unit that receives fluorescence emitted from the particles with irradiation with excitation light from the excitation optical system 20. The excitation optical system 20 and the light receiving optical system 30 constitute a fluorescence detection unit that detects fluorescence emitted from the particles collected on the substrate 10. The excitation optical system 20 and the light receiving optical system 30 carry out measurement of fluorescence emitted from the particles collected on the substrate 10 before and after the particles are heated.

The excitation optical system 20 includes a light emitting element 21 serving as a light source, an exciting-portion frame 22, a light collecting lens 24, a lens presser 25, and a filter 26. As the light emitting element 21, a semiconductor laser element for emitting blue laser light with a wavelength of 405 nm is used. Alternatively, an LED (Light Emitting Diode) may be used as the light emitting element 21. The wavelength of light emitted from the light emitting element 21 may be in an ultraviolet range or a visible range as long as the light excites biogenic particles to cause fluorescence emission.

The light receiving optical system 30 includes a metallic noise shield 36, a light receiving element 34, a filter 37, a light-receiving portion frame 33, a Fresnel lens 32, and a lens presser 31. As the light receiving element 34, a photodiode or an image sensor is used for example. Between the light receiving element 34 and the noise shield 36, an amplifying circuit 55 is disposed to amplify signals detected by the light receiving element 34.

A cleaning unit 96 removes particles from the principal surface 11 of the substrate 10. The cleaning unit 96 includes a brush 97 serving as a cleaner, and a brush fixing portion 98. The cleaning unit 96 is fixedly supported on the collection power-supply circuit 61.

The brush 97 is formed by a fiber assembly having conductivity. For example, the brush 97 is formed of carbon fiber. The fiber diameter of the fiber assembly that forms the brush 97 is preferably within a range of 0.05 to 0.2 mm. One end of the brush 97 is supported by the brush fixing portion 98, and the other end thereof is a free end hanging from the brush fixing portion 98. As the brush 51 moves relative to the substrate 10 with the free end of the brush 97 being in contact with the principal surface 11 of the substrate 10, particles are removed from the substrate 10.

The collector for removing particles from the substrate 10 is not limited to the brush 97. For example, the collector may be a wiper shaped like a flat plate in contact with the principal surface 11 of the substrate 10, or a nozzle for jetting air toward the principal surface 11 of the substrate 10.

The particle detector 1 further includes a holding member 80 on which the substrate 10 is mounted and held, and a driving unit 70 serving as a moving mechanism for moving the substrate 10. The driving unit 70 includes a rotary motor 71 to be rotationally driven, a motor holder 72 for holding the rotary motor 71, and a motor presser 73 for positioning the rotary motor 71.

The holding member 80 includes a rotary base 81. The rotary base 81 is formed of a resin material having low thermal conductivity. The rotary base 81 has a shaft hole 82. By inserting an output shaft of the rotary motor 71 in the shaft hole 82, the rotary motor 71 and the rotary base 81 are coupled to each other. Upon driving of the rotary motor 71, the rotary base 81 rotates (forward, in reverse) about the position of the shaft hole 82.

The holding member 80 includes an arm 83 extending away from a rotation shaft of the rotary base 81. The arm 83 extends in a radial direction orthogonal to the rotation shaft of the rotary base 81, and has a frame-shaped portion at a distal end thereof. The frame-shaped portion has a shape such as to be able to receive the substrate 10. The substrate 10 is mounted on the holding member 80 by being received in the frame-shaped portion provided at the distal end of the arm 83.

The particle detector 1 includes a heater 91 serving as a heating unit. The heater 91 is disposed on a back surface of the substrate 10, and heats particles collected on the principal surface 11 of the substrate 10. The heater 91 is stuck on the back surface of the substrate 10. The heater 91 moves together with the substrate 10 during rotation of the rotary base 81. To the heater 91, a plurality of lines, including a power supply line to the heater 91 and a signal line of a sensor incorporated in the heater 91, are connected. These lines are led out of the cabinet of the particle detector 1.

A position sensor 76 is disposed on a side surface of the lower cabinet 5. The substrate 10 is moved in the cabinet of the particle detector 1 by rotation of the rotary motor 71. The position sensor 76 is provided to detect the current position of the substrate 10.

### [Structure of Fluorescence Detecting Unit]

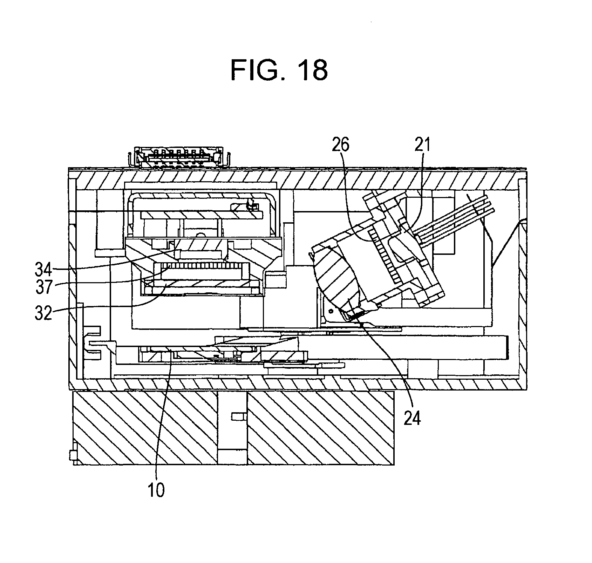

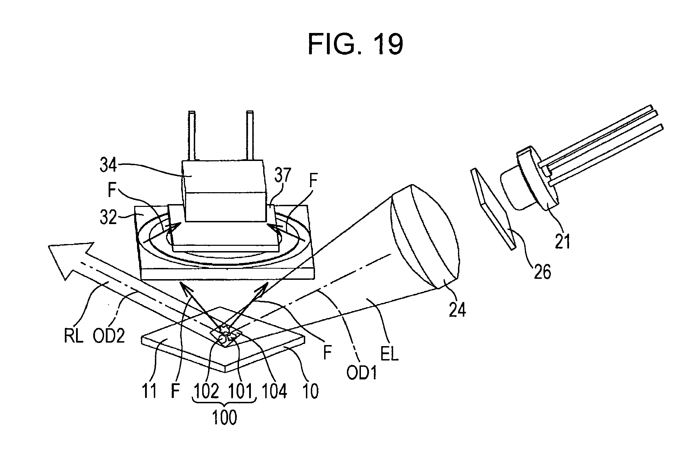

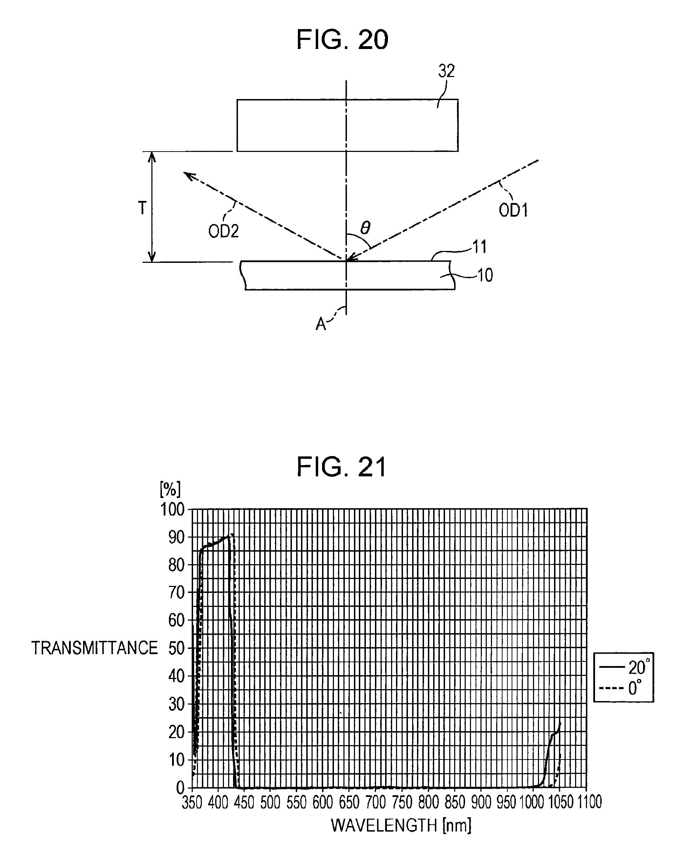

Fig. 18 is a cross-sectional view of the particle detector 1. Fig. 19 schematically illustrates the behavior of light in the optical system included in the particle detector 1. Fig. 20 schematically illustrates light reflection on the principal surface 11 of the substrate 10.

As described above, the excitation optical system 20 for irradiating the principal surface 11 of the substrate 10 with excitation light includes the light emitting element 21, the light collecting lens 24, and the filter 26.

It is only necessary that a cutoff wavelength λs of the filter 26 should be shorter than or equal to a cutoff wavelength λ1 in the filter function of an absorbent 32b contained in a below-described Fresnel lens 32 (λ1 ≥ λs). Preferably, the cutoff wavelength λs of the filter 26 is longer than or equal to 430 nm. Specifically, Fig. 21 is a graph showing a specific example of a filter characteristic of the filter 26, and shows the filter characteristic when an angle formed by the optical axis and the principal surface is 20° and 0°. The horizontal axis indicates the wavelength of light. The unit of the wavelength is nm. The vertical axis indicates the transmittance of the filter. The unit of the transmittance of the filter is %.

As shown in Fig. 21, for example, the filter 26 has the property of blocking most of light having a wavelength longer than or equal to about 430 nm (= λs) and transmitting most of light having a wavelength shorter than or equal to about 430 nm (= λs). The filter 26 functions as a low pass filter (or a high cut filter) for blocking light with a wavelength longer than or equal to a predetermined threshold value and transmitting light with a wavelength shorter than or equal to the threshold value.

When excitation light EL generated by the light emitting element 21 serving as a semiconductor laser element passes through the filter 26, it is not absorbed by the absorbent 32b contained in the below-described Fresnel lens 32, but a wavelength of about 500 nm or more to become a noise component is cut.

After passing through the filter 26, the excitation light EL is collected via the light collecting lens 24, and is applied onto an excitation-light irradiation region 104 on the principal surface 11 of the substrate 10. The excitation light EL obliquely enters the principal surface 11 of the substrate 10. In Figs. 19 and 20, a one-dot chain line denoted by symbol OD1 represents a ray direction of the excitation light EL. Here, the ray direction refers to a traveling direction of a luminous flux component of light (in this case, excitation light EL). In other words, the ray direction OD1 of the excitation light EL is an optical axis of the excitation optical system 20.

The principal surface 11 of the substrate 10 is a mirror surface. The excitation light EL incident on the principal surface 11 at an incident angle θ is specularly reflected by the principal surface 11. Light obtained by regular reflection of the excitation light EL on the principal surface 11 forms reflected light RL. In Figs. 19 and 20, a one-dot chain line denoted by symbol OD2 represents a ray direction of the reflected light RL. Since the excitation light EL is obliquely incident on the principal surface 11 of the substrate 10, the reflected light RL specularly reflected by the principal surface 11 is also obliquely reflected relative to the principal surface 11.

In Fig. 20, a one-dot chain line denoted by symbol A represents an optical axis of the light receiving optical system 30, that is, an optical axis of the Fresnel lens 32. The ray direction OD1 of the excitation light EL and the ray direction OD2 of the reflected light RL intersect the optical axis A. The ray directions OD1 and OD2 are at an angle to the optical axis A. The ray directions OD1 and OD2 are at an angle to an extending direction of the principal surface 11 of the substrate 10. Fig. 20 also shows a distance T between the principal surface 11 of the substrate 10 and the Fresnel lens 32.

Since the principal surface 11 is formed as a mirror surface, stray light is prevented from being caused by scattering of the excitation light EL on the principal surface 11. For this reason, it is possible to prevent the detection accuracy of the particle detector 1 from being reduced by the stray light as noise. When the principal surface 11 is a mirror surface, little scattering light is produced at reflection of the excitation light EL on the principal surface 11. Hence, interference due to stray light can be avoided by preventing only the reflected light RL, which is obtained from regular reflection of the excitation light EL by the principal surface 11, from being mixed into the light receiving optical system 30.

Further, since the filter 26 is provided, when cleaning residues are left by the brush 97 on the principal surface 11 of the substrate 10, the detection accuracy of the particle detector 1 can be prevented from being impaired by incidence, on the light receiving element 34, a component as noise that is not absorbed by the absorbent 32b contained in the below-described Fresnel lens 32, of excitation light EL scattered by irregularities on surfaces of the cleaning residues.

Particles 100 are collected in the excitation-light irradiation region 104. The particles 100 include biogenic particles 101 such as microorganisms, and non-biogenic dust 102 such as lint of chemical fiber. In Fig. 5, arrows denoted by symbol F represent fluorescence emitted from the particles 100. Fluorescence F is emitted in all directions from portions of surfaces of the particles 100 irradiated with excitation light EL. Fluorescence F traveling toward the light receiving optical system 30 is collected via the Fresnel lens 32, and is received by the light receiving element 34 via the filter 37. By using the Fresnel lens 32 as the light collecting lens for collecting the fluorescence F, the thickness of the light collecting lens can be reduced. This achieves reduction in size and weight of the particle detector 1.

The fluorescence F is emitted in all directions with no directivity from the particles 100 irradiated with the excitation light EL. Part of the fluorescence F directly travels from the particles 100 toward the Fresnel lens 32, and another part of the fluorescence F is emitted from the particles 100 toward the principal surface 11 of the substrate 10. When the principal surface 11 is a mirror surface, the fluorescence F is specularly reflected by the principal surface 11, and the reflected fluorescence F travels toward the Fresnel lens 32. Hence, the intensity of the fluorescence F collected by the Fresnel lens 32 and received by the light receiving element 34 can be increased. This can improve the detection sensitivity of the light receiving element 34 to the fluorescence F.

When the Fresnel lens 32 is disposed at a position at the distance T from the principal surface 11 of the substrate 10, the excitation light EL traveling toward the principal surface 11 of the substrate 10 and the reflected light RL regularly reflected by the principal surface 11 do not enter the Fresnel lens 32. Light that enters the Fresnel lens 32 is limited to only fluorescence F emitted from the particles 100, and neither the excitation light EL nor the reflected light RL is collected by the Fresnel lens 32. The light receiving optical system 30 is disposed at a position such that the excitation light EL and the reflected light RL do not enter the Fresnel lens 32 and the light receiving element 34 does not receive the excitation light EL and the reflected light RL. By doing this, the excitation light EL and the reflected light RL can be reliably separated from the fluorescence F, and the excitation light EL or the reflected light RL can be prevented from becoming noise in detection of the fluorescence F. Hence, the detection sensitivity to the fluorescence F can be improved.

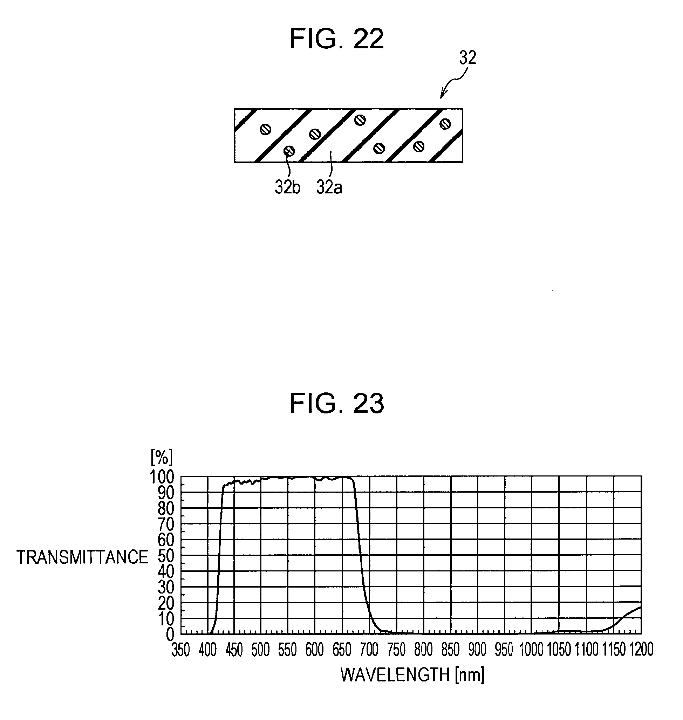

Fig. 22 schematically illustrates a cross section of the Fresnel lens 32. The Fresnel lens 32 is formed of a material containing a matrix 32a and absorbent 32b finely dispersed in the matrix 32a. The matrix 32a is formed of an arbitrary material that can be used as the Fresnel lens 32, and for example, may be formed of an acrylic material or a glass material that transmits fluorescence F. The absorbent 32b absorbs a light beam including a wavelength of excitation light EL. The absorbent 32b prevents the light beam including the wavelength of the excitation light EL from passing through the Fresnel lens 32. The Fresnel lens 32 has a filtering function for blocking the excitation light EL and the reflected light RL.

The absorbent 32b has the filter characteristic of the long pass filter shown by curve (1) of Fig. 13. That is, according to the filter characteristic shown by curve (1), the absorbent 32b has the property of blocking most of light with a wavelength shorter than or equal to about 440 nm and transmitting most of light with a wavelength longer than or equal to about 500 nm or more. The absorbent 32b has the property as a high pass filter (or a low cut filter) for blocking light with a wavelength shorter than or equal to a predetermined threshold value and transmitting light with a wavelength longer than or equal to the threshold value.

As shown by curve (2), excitation light EL from the semiconductor laser has the peak value of optical spectral intensity at a wavelength of about 400 nm, and the spectrum is not measured in the other wavelength ranges. In contrast, as shown by curve (3), the spectrum of fluorescence F from Penicillium is measured in a wavelength range longer than or equal to about 460 nm, and the fluorescence F has the peak value of optical spectral intensity at a wavelength of about 530 nm.

Since the absorbent 32b having the filter characteristic shown by curve (1) is dispersed in the Fresnel lens 32, when excitation light EL having the optical spectral intensity shown by curve (2) enters the Fresnel lens 32, it is absorbed and blocked by the absorbent 32b. In contrast, when the fluorescence F having the optical spectral intensity shown by curve (3) enters the Fresnel lens 32, it passes through the Fresnel lens 32 while mostly maintaining the optical spectral intensity.

By providing the Fresnel lens 32 with such a filter function, even if the excitation light EL or the reflected light RL erroneously enters the Fresnel lens 32, it can be blocked by the Fresnel lens 32. Thus, since a structure in which fluorescence F selectively passes through the Fresnel lens 32 is provided, noise is prevented from entering the light receiving element 34 for detecting the fluorescence F, and the detection sensitivity to the fluorescence F can be improved.

The filter 37 is an IR (infrared) cut filter, and it is only necessary that a cutoff wavelength λi thereof should be longer than or equal to the wavelength of infrared light (about 780 nm). Preferably, the cutoff wavelength λi is longer than or equal to 650 nm. Specifically, Fig. 23 is a graph showing a specific example of a filter characteristic of the filter 37. The horizontal axis indicates the wavelength of light. The unit of the wavelength is nm. The vertical axis indicates the transmittance of the filter. The unit of the transmittance of the filter is %.

As shown in Fig. 23, the filter 37 of this example has the property of blocking most of light with a wavelength longer than or equal to about 650 nm (= λi) and transmitting most of light with a wavelength longer than or equal to about 650 nm (= λi). That is, the filter 37 blocks light with a wavelength within an infrared region and transmits light with a wavelength shorter than or equal to the wavelength in the infrared region.

When light transmitted by the Fresnel lens 32 passes through the filter 37, an infrared component is cut from the light transmitted by the Fresnel lens 32, and the light enters the light receiving element 34. For this reason, when nitric acid or a nitric acid compound serving as cleaning residues are left by the brush 97 on the principal surface 11 of the substrate 10, the detection accuracy of the particle detector 1 can be prevented from being impaired by incidence of fluorescence as noise containing many infrared components that are generated by irradiation of the cleaning residues with excitation light EL.

### [Operation of Particle Detector]

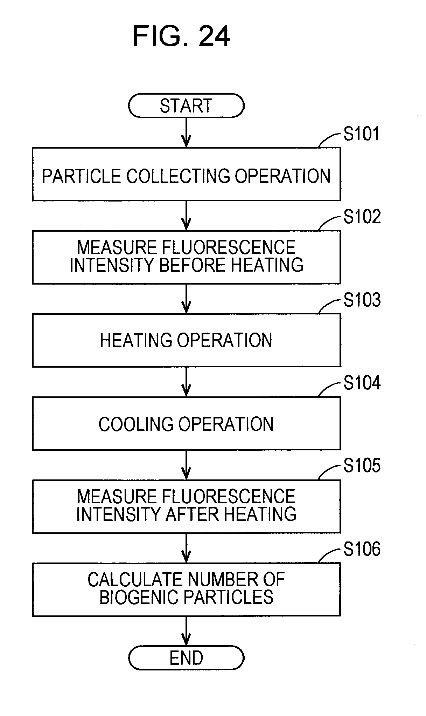

A description will be given of operations for detecting the number of biogenic particles 101 with the particle detector 1 having the above-described structure. Fig. 24 is a flowchart showing a flow of operations of the particle detector 1.

Referring to Fig. 24, first, particles 100 are collected on the principal surface 11 of the substrate 10 (S101). At this time, air is introduced into the cabinet of the particle detector 1 through the introducing portion 3 by driving the fan 92 in a forward direction to form an airflow traveling toward the principal surface 11 of the substrate 10. In addition, the electrostatic probe 62 is positioned opposed to the principal surface 11 of the substrate 10, and a potential difference is produced between the electrostatic probe 62 and the substrate 10 by the collection power-supply circuit 61. Particles 100 suspended in the air are thereby charged, and the charged particles 100 are collected onto the principal surface 11 of the substrate 10 by electrostatic force.

Next, the excitation optical system 20 applies excitation light EL to the particles 100 collected on the substrate 10, and the light receiving optical system 30 receives fluorescence F emitted from the particles 100 upon irradiation with the excitation light EL. The light emitting element 21 formed by the semiconductor laser element applies excitation light EL to the particles 100, and the light receiving element 34 receives fluorescence F emitted from the particles 100 at this time via the Fresnel lens 32. The fluorescence intensity before heating of the particles 100 collected on the substrate 10 is thereby measured (S102).

Next, the particles 100 collected on the substrate 10 are heated by energizing the heater 91 (S103). Next, energization of the heater 91 is stopped, and the substrate 10 is cooled (S104). At this time, air is introduced into the cabinet of the particle detector 1 through the fan 92 to promote cooling of the substrate 10 by driving the fan 92 in a reverse direction.

Next, the excitation optical system 20 applies excitation light EL onto the particles 100 collected on the substrate 10, and the light receiving optical system 30 receives fluorescence F emitted from the particles 100 upon irradiation with the excitation light EL. The fluorescence intensity after heating of the particles 100 collected on the substrate 10 is thereby measured (S105). By comparing the intensity of the fluorescence F before heating and the intensity of the fluorescence F after heating, the number of biogenic particles 101 included in the particles 100 collected on the substrate 10 is calculated (S106).

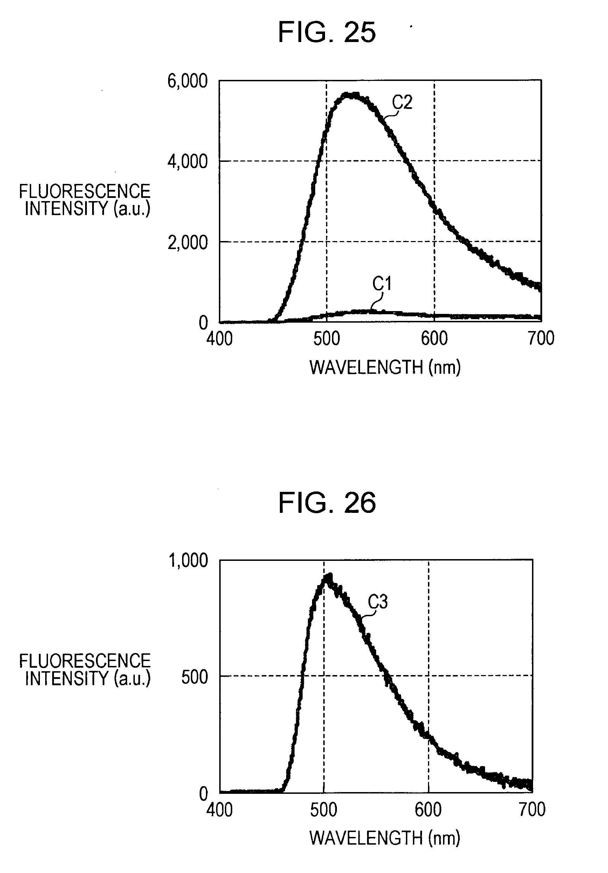

Fig. 25 shows the time change in fluorescence spectrum of Penicillium between before and after heat treatment. Fig. 25 shows measurement results of the fluorescence spectrum before heat treatment (curve C1) and after heat treatment (curve C2) when Penicillium is heated as examples of biogenic particles 101 at 200°C for five minutes. These results show that the intensity of fluorescence from the Penicillium is greatly increased by conducting heat treatment.

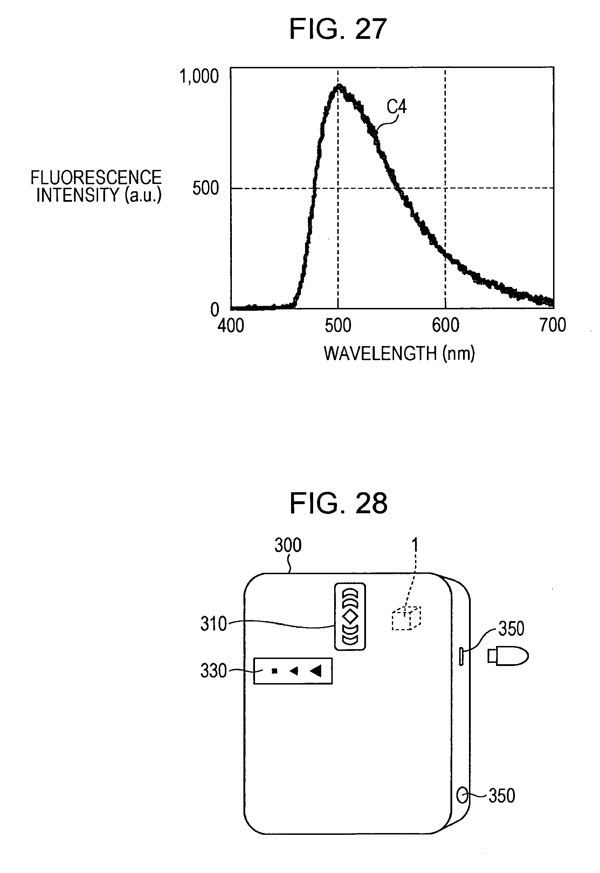

Fig. 26 shows the fluorescence spectrum of fluorescence-emitting dust before heat treatment. Fig. 27 shows the fluorescence spectrum of the fluorescence-emitting dust after heat treatment. Fig. 26 and Fig. 27 show measurement results of the fluorescent spectrum before heat treatment (curve C3) and after heat treatment (curve C4), respectively, when the fluorescence-emitting dust is heated at 200°C for five minutes. It is verified that the fluorescence spectrum shown by curve C3 and the fluorescence spectrum shown by curve C4 are nearly aligned with each other. That is, it is found that the intensity of fluorescence from the dust does not change between before and after heat treatment.

When the biogenic particles 101 suspended in the air are irradiated with ultraviolet light or blue light, they emit fluorescence F. However, dust 102 that similarly emits fluorescence, such as lint of chemical fiber, is suspended in the air. Hence, when only the fluorescence F is detected, it is not determined whether the fluorescence F is emitted from the biogenic particles 101 or the dust 102.

On the other hand, when the biogenic particles 101 and the dust 102 are subjected to heat treatment and changes in fluorescence intensity (fluorescence amount) between before and after heating are measured, the intensity of fluorescence emitted from the dust 102 is not changed by heat treatment, whereas the intensity of fluorescence emitted from the biogenic particles 101 is increased by heat treatment. In the particle detector 1 of the embodiment, the fluorescence intensity of the particles 100, in which the biogenic particles 101 and the dust 102 are mixed, is measured before and after heating, and a difference between the measured fluorescence intensities is found to specify the number of biogenic particles 101.

The intensity of fluorescence F emitted from the biogenic particles 101 is increased by heat treatment. For this reason, in Step (S105), a fluorescence intensity higher than the fluorescence intensity measured before heating in Step (S102) is measured. An increase amount in fluorescence intensity is calculated from the difference between the fluorescence intensity before heating and the fluorescence intensity after heating. On the basis of a prepared relationship between the increase amount in fluorescence intensity and the concentration of biogenic particles, the concentration of biogenic particles 101 corresponding to the calculated increase amount can be specified. The correspondence relationship between the increase amount and the concentration of biogenic particles is experimentally determined beforehand.

### [Configuration of Particle Removing Apparatus]

The particle detector 1 of the embodiment may be used alone as a device for detecting biogenic particles 101, or may be incorporated in home electric appliances such as an air purifier, an air conditioner, a humidifier, a dehumidifier, a vacuum cleaner, a refrigerator, and a television. Fig. 28 illustrates a specific example of an appearance of an air purifier 300 including the particle detector 1. The air purifier 300 is an example of a particle removing apparatus that efficiently removes biogenic particles 101 detected by the particle detector 1.

The air purifier 300 includes a switch 310 that receives operation instructions, and a display panel 330 that displays detection results and so on. The air purifier 300 also includes other unillustrated elements such as a suction opening through which air is introduced and an exhaust opening through which air is exhausted. The air purifier 300 further includes a communication unit 350 in which a recording medium is loaded. The communication unit 350 may provide connection to a communication line for communicating with other apparatuses through the Internet. Alternatively, the communication unit 350 may communicate with other apparatuses, for example, through infrared communication or through the Internet. The particle detector 1 is disposed within a housing of the air purifier 300. The air purifier 300 can efficiently purify ambient air by virtue of the particle detector 1 that can accurately detect biogenic particles 101 and can achieve size reduction.

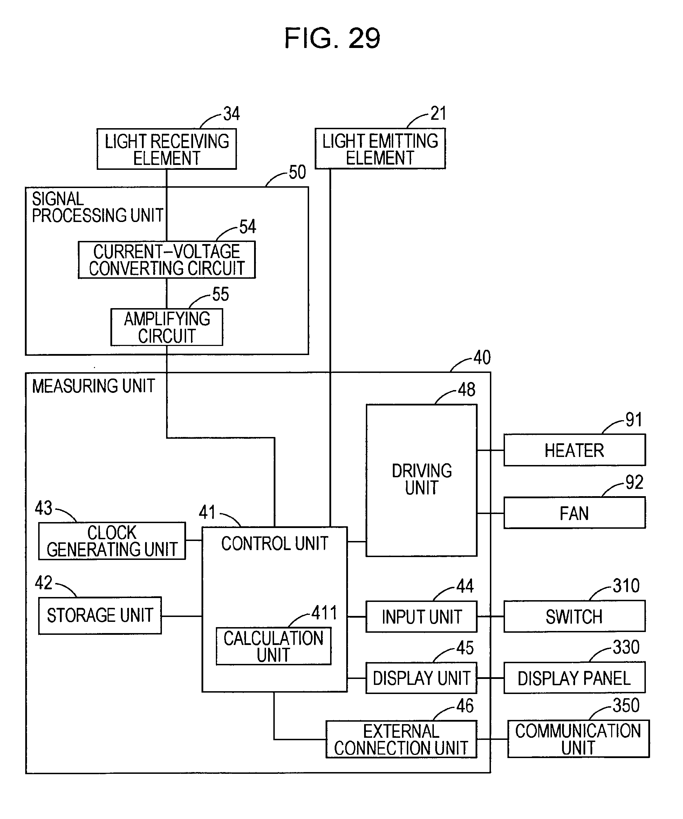

Fig. 29 is a block diagram of a specific example of a functional configuration of the air purifier 300. Fig. 29 illustrates an example in which the functions of a signal processing unit 50 are implemented by hardware configuration mainly of electric circuitry. However, at least part of these functions may be implemented by software configuration realized by an unillustrated CPU (Central Processing Unit) included in the signal processing unit 50 to execute a predetermined program. Further, Fig. 29 illustrates an example in which the function of a measuring unit 40 is implemented by software configuration. However, at least part of the functions may be realized by hardware configuration such as electric circuitry.

Referring to Fig. 29, the signal processing unit 50 includes a current-voltage converting circuit 54 connected to the light receiving element 34, and an integrating amplifying circuit 55 connected to the current-voltage converting circuit 54.

The measuring unit 40 includes a control unit 41, a storage unit 42, and a clock generating unit 43. The measuring unit 40 further includes an input unit 44 that receives input information by receiving an input signal from the switch 310 upon operation of the switch 310, a display unit 45 that executes processing for displaying, for example, measurement results on the display panel 330, an external connection unit 46 that executes processing necessary for exchanging data and the like with an external apparatus connected to the communication unit 350, and a driving unit 48 that drives the fan 92 and the heater 91.

When particles 100 collected on the principal surface 11 of the substrate 10 are irradiated with excitation light EL from the light emitting element 21, fluorescence F from the particles 100 in the excitation-light irradiation region 104 is collected at the light receiving element 34. The light receiving element 34 outputs a current signal in accordance with the amount of received light to the signal processing unit 50. The current signal is input to the current-voltage converting circuit 54.

The current-voltage converting circuit 54 detects a peak current value H, which represents the fluorescence intensity, from the current signal input from the light receiving element 34, and converts the peak current value H into a voltage value Eh. The voltage value Eh is amplified by a preset gain by the amplifying circuit 55, and is output to the measuring unit 40. The control unit 41 of the measuring unit 40 receives the input of the voltage value Eh from the signal processing unit 50, and stores the input in the storage unit 42 in order.

The clock generating unit 43 generates and outputs clock signals to the control unit 41. With the timing based on the clock signals, the control unit 41 outputs control signals for rotating the fan 92 to the driving unit 48, and controls introduction of air by the fan 92. Further, the control unit 41 is electrically connected to the light emitting element 21 and the light receiving element 34, and controls ON/OFF of these elements.

The control unit 41 includes a calculation unit 411. The calculation unit 411 performs operation for calculating the number of biogenic particles in the introduced air by using the voltage value Eh stored in the storage unit 42.

The concentration of the biogenic particles 101 in the collected particles 100, which is calculated by the calculation unit 411, is output from the control unit 41 to the display unit 45. The display unit 45 performs processing for displaying the input concentration of microorganisms on the display panel 330. For example, the display panel 330 has lamps corresponding to concentrations, and the display unit 45 specifies a lamp corresponding to the calculated concentration as a lamp to be lighted, and lights the lamp. As another example, a lamp may be lighted in different colors according to the calculated concentration. The display on the display panel 330 is not limited to lamp display. Numerical values, concentrations, or messages prepared beforehand corresponding to the concentrations may be displayed. The measurement results may be written on a recording medium loaded in the communication unit 350 or may be transmitted to an external apparatus via the communication unit 350 by the external connection unit 46.

The input unit 44 may receive selection of a display method on the display panel 330 according to an operation signal from the switch 310. Alternatively, the input unit 44 may receive selection of display of the measurement results on the display panel 330 or output of the measurement results to the external apparatus. A signal indicating the contents of selection is output to the control unit 41, and the control unit 41 outputs a necessary control signal to the display unit 45 and/or the external connection unit 46.

The particle detector 1 utilizes the difference in characteristic caused between fluorescence F from the biogenic particles 101 and fluorescence F from the dust 102 for emitting the fluorescence F by heat treatment, and detects biogenic particles 101 on the basis of an increase amount after predetermined heat treatment. For this reason, even when dust 102 for emitting fluorescence F is contained in the introduced air, the particle detector 1 accurately detects biogenic particles 101 separately from the fluorescence-emitting dust 102 on a real-time basis.

The air purifier 300 can efficiently remove the biogenic particles 101 by utilizing the concentration of the biogenic particles 101 detected by the particle detector 1 and by changing the operation state according to the output from the particle detector 1. That is, when the output of the particle detector 1 is large and the concentration of the biogenic particles 101 is high, a particle removing ability of the air purifier 300 can be enhanced, for example, by rotating the fan 92 at high speed to increase the ventilation amount. When the output of the particle detector 1 is small and the concentration of the biogenic particles 101 is low, the particle removing ability can be reduced. Hence, power-saving operation can be achieved by decreasing the rotation speed of the fan 92 to reduce the ventilation amount.

### [Experiment for Confirming Advantageous Effects of Embodiment]

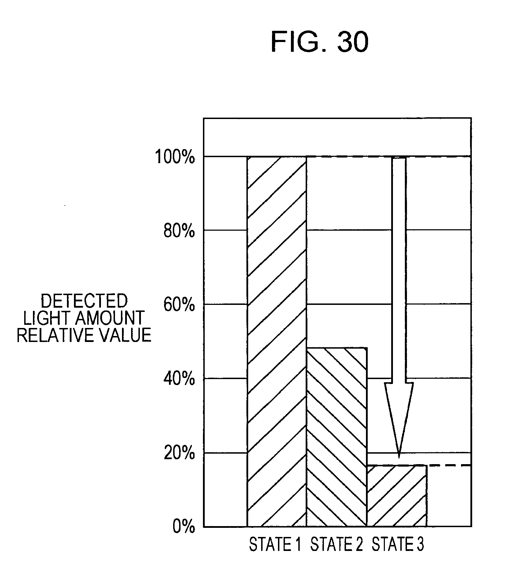

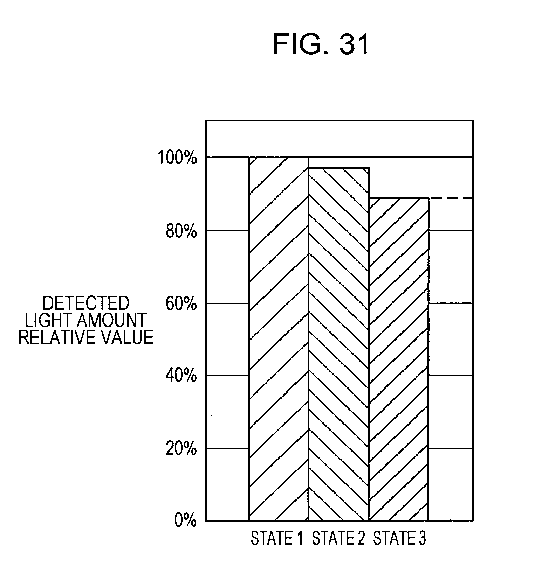

To confirm the advantageous effects obtained by disposing the filter 26 and the filter 37 in the particle detector 1, the amount of stray light and the fluorescence intensity in a state in which a predetermined amount of mold fungus was collected by the substrate 10 were measured in three states, that is, a state in which none of the filter 26 and the filter 37 were provided (state 1), a state in which only the filter 26 was provided, but the filter 37 was not provided (state 2), and a state in which both of the filters 26 and 37 were provided, and the results were compared. Figs. 30 and 31 show the results. The vertical axis of Fig. 30 indicates the amount of detected light. The amount of detected light refers to the relative value of the detected light amount in states 2 and 3 when the detected light amount in state 1 is 100. The vertical axis of Fig. 31 indicates the signal intensity corresponding to the fluorescence intensity. The signal intensity refers to the relative value of the signal intensity in states 2 and 3 when the signal intensity in state 1 is 100.

Specifically, referring to Fig. 30, the amount of stray light in state 2 is about 50% of the amount of stray light in state 1. This shows that the amount of stray light due to cleaning residues is reduced by about 50% by disposing the filter 26. Further, the amount of stray light in state 3 is about 20% of the amount of stray light in state 1. This shows that the amount of stray light due to cleaning residues is reduced by about 80% by disposing the filter 26 and the filter 37. Therefore, this experiment shows that disposition of the filter 26 has the effect of cutting scattering of excitation light due to irregularities of cleaning residues on the surface of the substrate. Also, the experiment shows that disposition of the filter 37 further increases this effect.

Referring to Fig. 31, the fluorescence intensity measured from the substrate 10 in state 2 is not greatly changed from state 1, but the fluorescence intensity measured from the substrate 10 in state 3 is about 90% of that in state 1. This shows that the fluorescence intensity measured from the substrate 10 is reduced by 10% or more by disposing the filter 37 and that fluorescence from the nitric acid and the nitric acid compound serving as cleaning residues are cut. Therefore, this experiment shows that disposition of the filter 26 has an effect of cutting fluorescence from the nitric acid compound and the like serving as cleaning residues. Also, the experiment shows that disposition of the filter 37 further increases this effect.

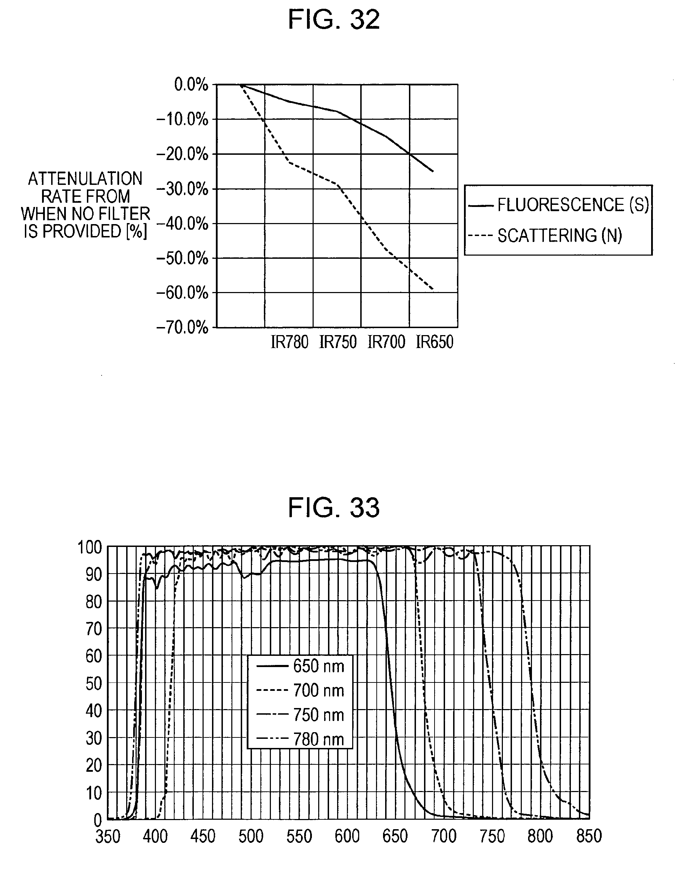

Further, the amount of stray light and the fluorescence intensity were measured while variously changing the cutoff wavelength of the filter 37. Fig. 32 shows the filter characteristics of IR filters used as the filter 37. Referring to Fig. 32, four types of IR filters having cutoff wavelengths of 650nm, 700 nm, 750 nm, and 780 nm were used in this experiment. The IR filter having the cutoff wavelength of 650 nm blocks light having a wavelength of about 650 nm or more, the IR filter having the cutoff wavelength of 700 nm blocks light having a wavelength of about 700 nm or more, the IR filter having the cutoff wavelength of 750 nm blocks light having a wavelength of about 750 nm or more, and the IR filter having the cutoff wavelength of 780 nm blocks light having a wavelength of about 780 nm or more.

Fig. 33 is a graph showing measurement results of the amount of stray light and the fluorescence intensity when these four types of filters are used as the filter 37. The vertical axis in the graph indicates the relative value when the filters are used in a case in which the value measured without using the IR filters is 100.

Referring to Fig. 33, both the amount of stray light and the fluorescence intensity become less than when the filter 37 is not provided, regardless of the type of the filter. For this reason, this experiment shows that fluorescence from the nitric acid compound and the like serving as cleaning residues and excitation light scattered by the cleaning residues on the surface of the substrate can be cut by disposing the filter 37. The experiment also shows that these effects are obtained when the cutoff wavelength λi of the filter 37 is longer than or equal to 650 nm. The experiment further shows that the decrease rate of the amount of stray light and the decrease rate of the fluorescence intensity increase, that is, the above effects further increase as the cutoff wavelength decreases.

It should be considered that the disclosed embodiment is not restrictive, but is illustrative in all respects. The scope of the present invention is defined by the claims, not by the above description. Further, the scope of the present invention is intended to include all modifications within the meaning and range equivalent to the scope of the claims.

### Reference Signs List

1: particle detector, 2: upper cabinet, 3: introducing portion, 4: collecting cylinder, 5: lower cabinet, 10: substrate, 11: principal surface, 20: excitation optical system, 21, 550: light emitting element, 22, 23: exciting-portion frame, 24: light collecting lens, 25, 31: lens presser, 26, 37: filter, 30: light receiving optical system, 32: Fresnel lens, 32a: matrix, 32b: absorbent, 33: light-receiving-portion frame, 34, 565: light receiving element, 36: noise shield, 40: measuring unit, 41: control unit, 42: storage unit, 43: clock generating unit, 44: input unit, 45: display unit, 46: external connection unit, 48, 70: driving unit, 50: signal processing unit, 54: voltage converting circuit, 55: amplifying circuit, 60: collecting unit, 61: collecting power-supply circuit, 62, 530: electrostatic probe, 71: rotary motor, 72: motor holder, 73: motor presser, 76: position sensor, 80: holding member, 81: rotary base, 82: shaft hole, 83: arm, 91, 520: heater, 97: brush, 92, 500: fan, 96: cleaning unit, 98: brush fixing portion, 100, 101, 600, 600A: particle, 102: dust, 104: excitation-light irradiation region, 300: air purifier, 310: switch, 330: display panel, 350: communication unit, 411: calculation unit, 510: collecting substrate, 560: lens, 600B: powder dust.

## Claims

1. A particle detector comprising:
a collecting member having a principal surface and configured to electrostatically collect particles on the principal surface;
an irradiation unit configured to irradiate the particles collected on the principal surface with excitation light;
a light receiving unit configured to receive fluorescence emitted from the particles by irradiation of the particles with the excitation light; and
a detection unit configured to detect biogenic particles from the particles collected on the principal surface on the basis of a fluorescence intensity in the light receiving unit,
wherein the particle detector further includes a filter disposed between the principal surface and the light receiving unit to cut light with a wavelength emitted by irradiation of the excitation light from a substance that is generated on the principal surface when the particles are electrostatically collected.

2. The particle detector according to Claim 1,
wherein the principal surface is a mirror surface, and
wherein the fluorescence is specularly reflected by the principal surface.

3. The particle detector according to Claim 1 or 2, further comprising:
a heating unit configured to heat the particles,
wherein the filter cuts light with a wavelength of fluorescence from a substance whose fluorescence amount changes between before and after heating with the heating unit, of the substance generated when the particles are electrostatically collected.

4. The particle detector according to any of Claims 1 to 3, wherein the filter cuts light with a wavelength of fluorescence from a nitric acid compound.

5. The particle detector according to any of Claims 1 to 4, wherein a cutoff wavelength λi of the filter is longer than or equal to 650 nm.

6. The particle detector according to any of Claims 1 to 5,
wherein the light receiving unit includes a light collecting lens, and
wherein the light collecting lens contains a material that absorbs the excitation light.

7. An air conditioner comprising the particle detector according to any of Claims 1 to 6,
wherein the air conditioner includes a display unit, and displays, on the display unit, a detection result of a number of particles in the air detected by the particle detector.
